# EUROPÄISCHE PATENTSCHRIFT

(11) **EP 0 317 811 B1**
(45) Veröffentlichungstag und Bekanntmachung des Hinweises auf die Patenterteilung: **03.03.1993**
(21) Anmeldenummer: 88118432.9
(22) Anmeldetag: 04.11.1988
(51) Int. Cl.: C12M 1/06, C12M 3/02

(54) **Fermenter zum Züchten von Zellkulturen**
Fermentor for growing cell cultures
Fermenteur pour cultures cellulaires

(30) Priorität: 23.11.1987 DE 3739650
(43) Veröffentlichungstag der Anmeldung: 31.05.1989
(73) Patentinhaber: IMMUNO Aktiengesellschaft, A-1221 Wien (AT)
(72) Erfinder: Mundt, Wolfgang, Dr., A-1080 Wien (AT)
(74) Vertreter: Grünecker, Kinkeldey, Stockmair & Schwanhäusser Anwaltssozietät

(56) Entgegenhaltungen:
- WO-A-85/02195
- WO-A-87/05322
- FR-A- 1 604 693
- FR-A- 2 393 535
- FR-A- 2 559 500
- SCIENTIFIC AMERICAN, Band 248, Nr. 1, Januar 1983, Seiten 24-31, New York, US; J. FEDER et al.: "The large-scale cultivation of mammalian cells"

## Beschreibung

Die Erfindung bezieht sich auf einen Fermenter zum Züchten von, insbesondere auf Trägerkörpereinheiten befindlichen, Zellkulturen, mit einem Gefäß, in dem die Zellkulturen mittels eines Rührwerks in Suspension haltbar sind, und mit einer Belüftungseinrichtung, mittels derer der Suspension fein verteilt Sauerstoff mit Abstand exzentrisch zur Rührachse zuführbar ist.

Zum Züchten werden Zellkulturen regelmäßig auf Trägerkörperchen, sogenannte Microcarrier, aufgebracht, mit denen sie in einer im Fermenter enthaltenen Flüssigkeit suspendiert werden. Um das Wachstum der Zellkulturen zu ermöglichen, wird der Suspension Sauerstoff, zumeist in Form von Luft, zugeführt.

Ursprünglich hat man die Luft einfach im unteren Bereich des Fermenters zugeführt. Die aufsteigenden Luftblasen haben jedoch zur Schaumbildung und damit zur Zerstörung der Zellen auf den oberen Trägerkörperchen geführt.

Um die Zerstörung der Zellkulturen zu vermeiden, wurde dann eine passive Sauerstoffanreicherung der Suspension versucht, indem man das Fermentergefäß kegelstumpfförmig ausgebildet hat, so daß die der Umgebungsluft ausgesetzte Oberfläche der Suspension vergrößert wurde. Die Sauerstoffaufnahme bei diesem Verfahren ist jedoch unzureichend.

Auch frühere Versuche, die Sauerstoffanreicherung in getrennten Gefäßen durchzuführen oder den Sauerstoff durch ein Sieb in die Suspension einzublasen, haben nicht immer den erwünschten Erfolg gehabt.

Ausgehend von dem zuletzt genannten Versuch hat man dann zentral in das Gefäß des Fermenters einen zylindrischen Siebkorb eingebaut, in dem auf einer axial schwingenden Achse Prallplatten angebracht sind, die konische Durchgangsbohrungen aufweisen. Die konischen Durchgangsbohrungen sind auf jeweils einer Platte gegenläufig angeordnet, so daß durch die Schwingbewegungen der Platten Pumpbewegungen von einer konischen Bohrung zur benachbarten konischen Bohrung entstehen. Der in den Siebkorb durch die Achse eingeführte Sauerstoff wird aufgrund der entstehenden Scherströmungen sehr fein verteilt und fast gelöst. Die Schaumbildung, die bei den früheren Verfahren zu beobachten war, ist damit vermieden. Jedoch ist die Sauerstoffaufnahme der Suspension immer noch zu gering. Außerdem ist es nunmehr erforderlich, daß das in jedem Fermenter vorhandene Rührwerk von unten angetrieben werden muß. Hierdurch bedingt muß eine Welle durch den Boden des Gefäßes hindurchgeführt werden, wodurch sich Dichtungsprobleme ergeben und wodurch es insbesondere zu einem Stau von Trägerkörperchen zwischen der Mitte des Rührflügels des Rührwerks und dem Gefäßboden kommt, was zu Zerstörung von Zellkulturen führt.

Schließlich ist aus der DE 35 04 748 C2 ein Fermenter der eingangs beschriebenen Art bekannt, bei dem die Belüftungseinrichtung aus einem Luftrohr mit einem Filter und einem porösen Auslaßkörper besteht, der unmittelbar in die Suspension mündet. Der poröse Mündungskörper ist zwischen der Gefäßwand und der äußeren Umlaufbahn des Rührwerks, etwas an letztere angenähert, angeordnet. Ein solcher direkter Eintrag des Sauerstoffs in die Suspension ist praktisch nur möglich, wenn die Zellen abgekapselt sind. Bei dem bekannten Fermenter werden deswegen kugelförmige Kapseln mit einer semipermeablen Membran verwendet, in denen sich die Zellen befinden. Bei in Kapseln enthaltenen Zellen kann das Rührwerk mit einer so hohen Drehzahl laufen, daß die unmittelbar in die Suspension eingebrachten Gasbläschen zerschlagen werden und der Sauerstoff dadurch gleichmäßig in der Suspension verrührt wird. Bei dem Fermenter nach der DE 35 04 748 ist die Belüftungseinrichtung nur deswegen exzentrisch außerhalb des Rührwerks angeordnet, weil im Zentrum des Gefäßes, wegen des dort befindlichen Rührwerks, kein Platz für die Belüftungseinrichtung vorhanden ist.

Der Erfindung liegt die Aufgabe zugrunde, einen Fermenter zu schaffen, in welchem mit einfachen baulichen Mitteln auch sehr empfindliche Zellkulturen zu intensivem Wachstum gebracht werden können.

Diese Aufgabe wird bei einem Fermenter der eingangs erläuterten Art dadurch gelöst, daß die Belüftungseinrichtung am Rand des Gefäßes im Bereich höherer Strömungsgeschwindigkeit der Flüssigkeit angeordnet und als Siebbelüfter mit einem zu einem Hohlkörper geformten Sieb, dem durch eine zentrale Achse der Sauerstoff zuführbar ist, ausgebildet ist.

Bei dem erfindungsgemäßen Fermenter wird das sauerstoffhaltige Gas über einen Siebbelüfter, also indirekt, der Suspension zugeführt. In dem durch das Sieb gebildeten Hohlkörper befinden sich keine Zellkulturen, so daß eine Schaumbildung nicht zu befürchten ist. Durch die exzentrische Anordnung des Siebbelüfters bzw. der Siebbelüfter in der Nähe des Gefäßrandes wird der Sauerstoffeintrag in die Suspension in überraschend hohem Maße gesteigert. Am Orte der Siebbelüfteranordnung befindet sich die durch das Rührwerk in Rotation versetzte Suspension nämlich in einem Zustand hoher Fließgeschwindigkeit, so daß die mit Sauerstoff angereicherte Flüssigkeit aus jedem Hohlkörper-Sieb rasch herausgespült und mit der übrigen Suspension vermischt wird. Damit ist das bisher bei Siebbelüftern aufgetretene Problem beseitigt, daß innerhalb des Siebes sehr rasch eine Sauerstoffsättigung eintritt, so daß auch durch erhöhte Sauerstoffzufuhr zum Sieb kein stärkerer Sauerstoffeintrag erreicht werden kann. Mit der Erfindung wird also gezielt die beim Rühren in Richtung zum Gefäßrand zunehmende Bewegungsgeschwindigkeit der Suspension ausgenutzt, um den indirekten Sauerstoffeintrag über ein Sieb zu verstärken, was gelingt, obwohl wegen der Empfindlichkeit der Zellkulturen das Rührwerk nur sehr langsam laufen darf.

Zweckmäßigerweise ist vorgesehen, daß in dem Sieb übereinander auf einer mit einem Vibrationsantrieb verbundenen Schwingachse Prallplatten mit mehreren konischen Durchgangsbohrungen angeordnet sind, und daß die Schwingachse zum Zuführen des Sauerstoffs hohl ausgebildet ist. Auf diese Weise wird eine besonders intensive Sauerstoffeinbringung ohne Schaumbildung gewährleistet.

Die Porenweite des Siebes soll etwa 100 µm betragen.

Bei größeren Fermentern können zwei oder mehr Belüftungseinrichtungen über den Umfang des Gefäßes verteilt vorgesehen sein.

Dichtungsprobleme werden dadurch günstigerweise vermieden, daß die Antriebswelle des Rührwerks von oben in das Gefäß hineinragt.

Der Sauerstoffpartialdruck der Suspension kann dadurch ständig überwacht werden, daß in dem Gefäß ein Sensor zum Messen des Sauerstoffpartialdruckes angeordnet ist.

Auf diese Weise läßt sich auch eine genaue Steuerung der Sauerstoffeintragung bewirken, weil der Sensor mit einer Steuereinrichtung für die Sauerstoffabgabe verbunden werden kann.

Bei mehreren Belüftungseinrichtungen können diese wahlweise abschaltbar sein, wodurch eine einfache Steuerungsmöglichkeit für den Sauerstoffeintrag gegeben ist.

Die Steuerung kann dann nämlich dadurch erfolgen, daß die Belüftungseinrichtungen in Abhängigkeit des gemessenen Sauerstoffpartialdruckes an- und abschaltbar sind.

Im folgenden wird ein Ausführungsbeispiel der Erfindung anhand einer Zeichnung näher erläutert. Es zeigen:
- Fig. 1: schematisch in einem Vertikalschnitt ein Ausführungsbeispiel eines erfindungsgemäßen Fermenters,
- Fig. 2: den Fermenter aus Fig. 1 gesehen in Ansicht II, und
- Fig. 3: ein Detail III des Fermenters aus Fig. 1.

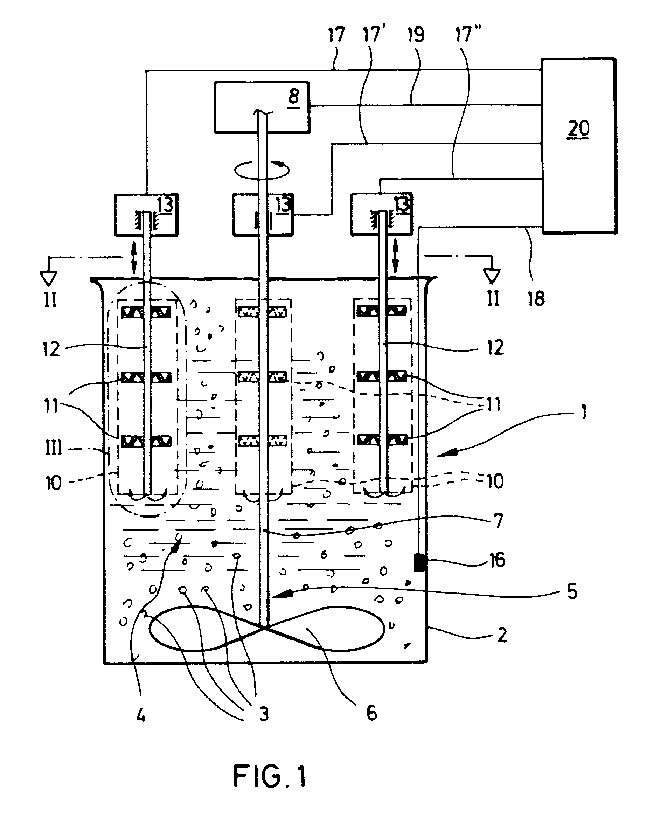

Die Fig. 1 zeigt einen Fermenter 1 mit einem oben geschlossenem Gefäß 2, in dem Zellkulturen auf Trägerkörperchen 3, sogenannten Microcarriern, in einer Flüssigkeit 4 in Suspension gehalten werden. Es handelt sich bei diesem Ausführungsbeispiel um Kunststoffkügelchen als Trägerkörperchen.

Der Fermenter 1 weist ein Rührwerk 5 mit einem Rührflügel 6, einer Antriebswelle 7 und einem Motor 8 auf. Die Antriebswelle 7 fällt mit der Rührachse, um die sich die durch das Rührwerk 5 verursachte Drehströmung dreht, zusammen.

Darüber hinaus umfaßt der Fermenter 1 bei dem hier gezeigten Ausführungsbeispiel vier Belüftungseinrichtungen 9.

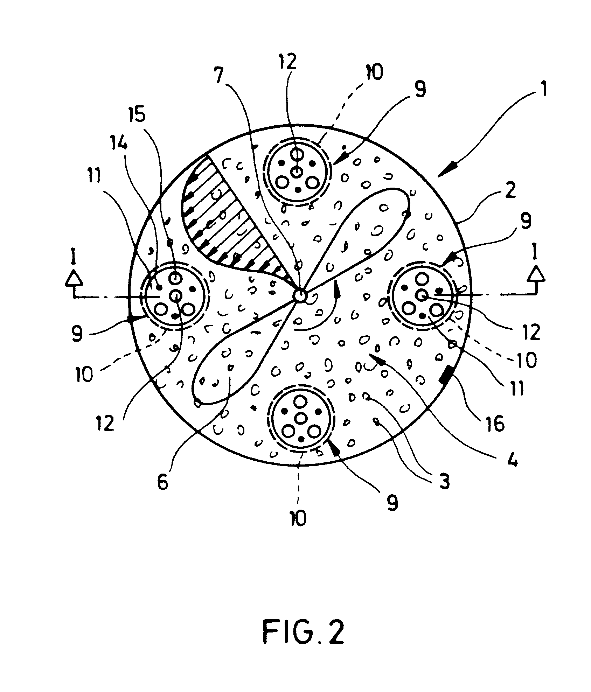

Wie besonders gut aus Fig. 2 ersichtlich ist, sind die Belüftungseinrichtungen 9 mit Abstand exzentrisch zur Rührachse (Antriebswelle 7) angeordnet. Die Belüftungseinrichtungen 9 sind nahe am Rand des Gefäßes 2 angebracht.

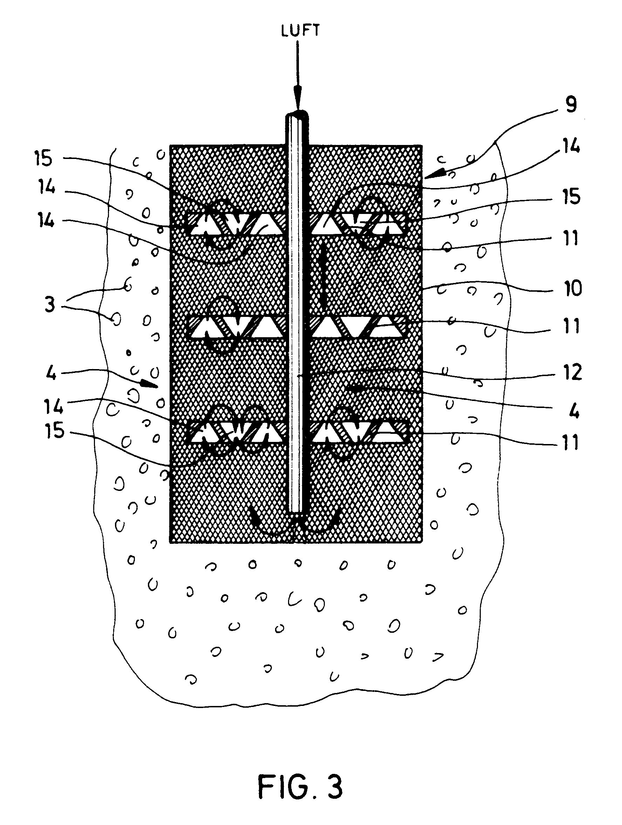

Wie besonders gut aus den Fig. 1 und 3 ersichtlich ist, sind die Belüftungseinrichtungen als sogenannte Siebbelüfter ausgebildet. Hierzu weist eine solche Belüftungseinrichtung 9 ein zu einem zylindrischen Hohlkörper geformtes Sieb 10 auf, in dem übereinander mehrere Prallplatten 11 angeordnet sind. Die Prallplatten 11 sind über eine Schwingachse 12, die axial zu dem Sieb 10 angeordnet ist, miteinander verbunden. Die Schwingachse 12 ist an ihrem oberen Ende in einem Vibrationsantrieb 13 gelagert, der die Schwingachse in axialer Richtung in hochfrequente Schwingungen versetzt. Die Schwingachse 12 überträgt diese Vibrationen auf die Prallplatten 11.

Wie aus Fig. 3 deutlich erkennbar ist, weisen die einzelnen Prallplatten mehrere konische Durchgangsbohrungen 14 und 15 auf. Die Durchgangsbohrungen 14 und 15 sind so angeordnet, daß benachbarte Bohrungen 14 und 15 gegeneinander gerichtet sind, d.h. die eine Durchgangsbohrung 14 verjüngt sich nach oben, während sich die benachbarte Durchgangsbohrung 15 nach unten verjüngt.

Die Schwingachsen 12 sind im Inneren hohl ausgebildet und unten offen. Der von der Belüftungseinrichtung 9 in die Flüssigkeit zu mischende Sauerstoff gelangt in Pfeilrichtung durch das Innere der Schwingachsen 12 hindurch und tritt am unteren Ende aus. Die nach oben steigenden Luftblasen werden durch die Prallplatten fein verteilt. Der übersichtlichkeit halber sind die Luftventile und die Luftfördereinrichtung nicht dargestellt.

Die Porenweite des Siebes 10 beträgt bei dem hier gezeigten Ausführungsbeispiel etwa 100 µm.

In dem Gefäß 2 ist ein Sensor 16 zum Messen des Sauerstoffpartialdruckes der Suspension angeordnet. Die Vibrationsantriebe 13, der Motor 8 und der Sensor 16 sind über Steuerleitungen 17, 17′, 17˝, ..., sowie 18 und 19 mit einer Steuereinrichtung 20 verbunden. Mit der Steuereinrichtung 20 können der Motor 8, die Vibrationsantriebe 13 und die nicht dargestellten Luftventile zum Einleiten der Luft durch die Schwingachsen 12 ein- und ausgeschaltet werden. Darüber hinaus läßt sich die Anzahl der in Betrieb befindlichen Belüftungseinrichtungen in Abhängigkeit des von dem Sensor 16 gemessenen Sauerstoffpartialdrucks steuern.

Im folgenden wird ein Ausführungsbeispiel des erfindungsgemäßen Verfahrens anhand des oben beschriebenen Fermenters näher erläutert.

In dem Gefäß 2 befindet sich die Flüssigkeit 4, in der Zellkulturen auf Trägerkörperchen 3 vorliegen. Das Rührwerk 5 wird in Bewegung gesetzt, so daß sich der Rührflügel 6 entgegen dem Uhrzeigersinn dreht und die Trägerkörperchen 3 in Suspension in der Flüssigkeit 4 hält (vgl. Fig. 2).

Um die Rührachse (Antriebswelle 7) des Rührwerkes 5 bildet sich eine Drehströmung aus. In Fig. 2 ist andeutungsweise das Strömungsprofil dieser Drehströmung dargestellt. Es ist zu erkennen, daß in der Nähe des Randes des Gefäßes 2 eine wesentlich höhere Strömungsgeschwindigkeit herrscht, als im Zentrum des Gefäßes 2, wo die Strömungsgeschwindigkeit vernachlässigbar ist. Zur Belüftung der Suspension in dem Fermenter 1 wird über die Belüftungseinrichtungen 9 Sauerstoff eingebracht und zwar im Bereich der größten Strömungsgeschwindigkeit der oben angesprochenen Drehströmung.

Zum Einbringen von Sauerstoff in die Suspension werden je nach gewünschtem Partialdruck ein oder mehrere Belüftungseinrichtungen 9 in Betrieb gesetzt. Die nicht dargestellten Luftventile lassen also Luft bzw. Sauerstoff durch die Schwingachsen in das Innere der Siebe 10 einströmen. Die Prallplatten 11 werden mittels der Vibrationsantriebe 13 auf- und abbewegt. Im Inneren des Siebes 10 befindet sich nur Flüssigkeit 4, aber kein Trägerkörperchen 3. Innerhalb der Siebe können daher hohe Scherströmungen auftreten, ohne daß Zellkulturen auf den Trägerkörperchen 3 zerstört werden. Diese hohen Scherströmungen werden erzeugt durch die hochfrequente Auf- und Abbewegung der Prallplatten und die durch die konischen Durchgangsbohrungen 14 und 15 erzeugte Pumpströmung. Die in die Belüftungseinrichtung 9 gelangende Luft wird auf diese Weise gut verteilt. Die Luftbläschen werden so klein "zerhackt", daß die Luft in der Flüssigkeit 4 gelöst wird. Die so in der Flüssigkeit 4 gelöste Luft wird aufgrund dessen, daß die Belüftungseinrichtungen im Bereich einer verhältnismäßig schnellen Strömung liegen, schnell unter die Suspension außerhalb der Belüftungseinrichtungen 9 gemischt.

Auf diese Weise kann stets eine optimale Sauerstoff-Perfusion erreicht werden.

## Patentansprüche

1. Fermenter zum Züchten von, insbesondere auf Trägerkörpereinheiten befindlichen Zellkulturen, mit einem Gefäß, in dem die Zellkulturen mittels eines Rührwerks in Suspension haltbar sind, und mit einer Belüftungseinrichtung, mittels derer der Suspension fein verteilt Sauerstoff mit Abstand exzentrisch zur Rührachse zuführbar ist, **dadurch gekennzeichnet,** daß die Belüftungseinrichtung (9) am Rand des Gefäßes (2) im Bereich höherer Strömungsgeschwindigkeit der Flüssigkeit angeordnet und als Siebbelüfter mit einem zu einem Hohlkörper geformten Sieb (10), dem durch eine zentrale Achse (12) der Sauerstoff zuführbar ist, ausgebildet ist.

2. Fermenter nach Anspruch 1, **dadurch gekennzeichnet,** daß in dem Sieb (10) übereinander auf einer mit einem Vibrationsantrieb (13) verbundenen Schwingachse (12) Prallplatten (11) mit mehreren konischen Durchgangsbohrungen (14, 15) angeordnet sind, und daß die Schwingachse (12) zum Zuführen des Sauerstoffs hohl ausgebildet ist.

3. Fermenter nach Anspruch 1 und/oder 2, **dadurch gekennzeichnet,** daß die Porenweite des Siebes (10) etwa 100 µm beträgt.

4. Fermenter nach den Ansprüchen 1 bis 3, **dadurch gekennzeichnet,** daß zwei oder mehr Belüftungseinrichtungen (9) über den Umfang des Gefäßes (2) verteilt vorgesehen sind.

5. Fermenter nach den Ansprüchen 1 bis 4, **dadurch gekennzeichnet,** daß das die Antriebswelle (7) des Rührwerkes (5) von oben in das Gefäß (2) hineinragt.

6. Fermenter nach den Ansprüchen 1 bis 5, **dadurch gekennzeichnet,** daß in dem Gefäß (2) ein Sensor (16) zum Messen des Sauerstoffpartialdruckes der Suspension angeordnet ist.

7. Fermenter nach den Ansprüchen 1 bis 6, **dadurch gekennzeichnet,** daß der Sensor (16) mit einer Steuereinrichtung (20) zum Steuern der Sauerstoffabgabe der Belüftungseinrichtung (9) verbunden ist.

8. Fermenter nach den Ansprüchen 1 bis 7, **dadurch gekennzeichnet,** daß bei mehreren Belüftungseinrichtungen (9) diese wahlweise abschaltbar sind.

9. Fermenter nach einem der Ansprüche 1 bis 8, **dadurch gekennzeichnet,** daß die Belüftungseinrichtungen (9) in Abhängigkeit des gemessenen Sauerstoffpartialdrucks an- und abschaltbar sind.

## Claims

1. Fermenter for growing, in particular, cell cultures located on support bodies, having a vessel in which the cell cultures can be kept in suspension by means of a stirrer and having an aeration device, by means of which finely divided oxygen can be fed to the suspension at a distance eccentric to the stirrer axis, characterised in that the aeration device (9) is arranged at the edge of the vessel (2) in the region of relatively high flow velocity of the liquid and is designed as a sieve aerator having a sieve (10) shaped as a hollow body, to which the oxygen can be fed through a central axis (12).

2. Fermenter according to Claim 1, characterised in that baffle plates (11) having a plurality of conical through-holes (14, 15) are arranged in the sieve (10) one above the other on a swing axle (12) connected to a vibration drive (13), and in that the swing axle (12) is designed to be hollow for feeding the oxygen.

3. Fermenter according to Claim 1 and/or 2, characterised in that the pore width of the sieve (10) is about 100 µm.

4. Fermenter according to Claims 1 to 3, characterised in that two or more aeration devices (9) are provided distributed about the periphery of the vessel (2).

5. Fermenter according to Claims 1 to 4, characterised in that the drive shaft (7) of the stirrer (5) projects into the vessel (2) from above.

6. Fermenter according to Claims 1 to 5, characterised in that a sensor (16) for measuring the partial pressure of the oxygen in the suspension is arranged in the vessel (2).

7. Fermenter according to Claims 1 to 6, characterised in that the sensor (16) is connected to a control device (20) for controlling the oxygen delivery of the aeration device (9).

8. Fermenter according to Claims 1 to 7, characterised in that where a plurality of aeration devices (9) are present, these can be selectively turned off.

9. Fermenter according to one of Claims 1 to 8, characterised in that the aeration devices (9) can be turned on and off depending on the measured partial pressure of the oxygen.

## Revendications

1. Fermenteur pour faire croître des cultures cellulaires se trouvant en particulier sur des corpuscules porteurs, comportant un récipient dans lequel les cultures cellulaires peuvent être maintenues en suspension à l'aide d'un agitateur et comportant un dispositif d'aération au moyen duquel on peut amener à la supension à une certaine distance excentriquement par rapport à l'axe de l'agitateur, de l'oxygène finement divisé, fermenteur caractérisé par le fait que le dispositif d'aération (9) est disposé au bord du récipient (2), dans la zone de la plus grande vitesse d'écoulement du liquide et qu'il est conçu sous forme d'un aérateur à toile de tamisage présentant une toile de tamisage (10) qui est mise en forme de corps creux dans lequel on peut amener l'oxygène par un axe central (12).

2. Fermenteur selon la revendication 1, caractérisé par le fait que dans la toile de tamisage (10) sont disposées, l'une au-dessus de l'autre sur un axe vibrant (12) lié à un mécanisme d'entrainement en vibration (13), des plaques à chicanes (11) présentant plusieurs perçages traversants coniques (14, 15), et par le fait que l'axe vibrant (12) est de forme creuse pour amener l'oxygène.

3. Fermenteur selon la revendication 1 et/ou la revendication 2, caractérisé par le fait que la largeur de maille de la toile de tamisage (10) vaut environ 100 µm.

4. Fermenteur selon la revendication 1 à 3, caractérisé par le fait qu'il est prévu deux ou plus dispositifs d'aération (9), répartis sur la périphérie du récipient (2).

5. Fermenteur selon les revendications 1 à 4, caractérisé par le fait que l'arbre d'entrainement (7) de l'agitateur (5) pénètre dans le récipient (2) de par en haut.

6. Fermenteur selon les revendications 1 à 5, caractérisé par le fait que dans le récipient (2) est disposé un détecteur (16) pour mesurer la pression partielle d'oxygène dans la suspension.

7. Fermenteur selon les revendications 1 à 6, caractérisé par le fait que le détecteur (16) est relié à un dispositif de commande (20) pour commander l'émission d'oxygène du dispositif d'aération (9).

8. Fermenteur selon les revendications 1 à 7, caractérisé par le fait que dans le cas de plusieurs dispositifs d'aération (9), ceux-ci peuvent être mis hors circuit à volonté.

9. Fermenteur selon l'une des revendications 1 à 8, caractérisé par le fait que les dispositifs d'aération (9) peuvent être mis en circuit et hors circuit en fonction de la pression partielle d'oxygène mesurée.
